# EUROPEAN PATENT SPECIFICATION

(11) **EP 1 163 902 B1**
(45) Date of publication and mention of the grant of the patent: **17.01.2007**
(21) Application number: 01114504.2
(22) Date of filing: 15.06.2001
(51) Int. Cl.: A61K 9/70

(54) **Transdermal preparation containing hydrophilic or salt-form drug**
Einen hydrophilen oder als Salz vorliegenden Arzneiwirkstoff enthaltende transdermale Zubereitung
Préparation transdermique contenant un principe actif hydrosoluble ou sous forme de son sel

(30) Priority: 16.06.2000 KR 2000033330
(43) Date of publication of application: 19.12.2001
(73) Proprietor: PACIFIC CORPORATION, Seoul 140-777 (KR)
(72) Inventor: Lee, Wan Suk, Seongnam-si, 463-010 Kyunggi-do (KR); Lee, Young Dae, Yongin-si, 449-900 Kyunggi-do (KR); Kim, Jung Ju, Suji-eup, Yongin-si, 449-840 Kyunggi-do (KR)
(74) Representative: Barz, Peter

(56) References cited:
- US-A- 5 035 894
- US-A- 5 505 956
- HUANG Y ET AL: "Molecular aspects of muco- and bioadhesion: - Tethered structures and site-specific surfaces" JOURNAL OF CONTROLLED RELEASE, ELSEVIER SCIENCE PUBLISHERS B.V., AMSTERDAM, NL, vol. 65, no. 1-2, March 2000 (2000-03), pages 63-71, XP004190312 ISSN: 0168-3659

## Description

### FIELD OF THE INVENTION

The present invention relates to a transdermal preparation, and more specifically, relates to a transdermal preparation with improved skin permeability, which contains higher concentration of hydrophilic or salt-form drug.

### BACKGROUND OF THE INVENTION

Recently, development of new drug delivery system starts to be spotlighted and occupies the majority of pharmaceutical sciences. Objective of the development of drug delivery system is to provide effectiveness and safety of drug administration and patient convenience. Among the various drug delivery systems, the development of transdermal drug delivery system based on change of administration route plays a leading role. Preparations based on the transdermal drug delivery system using, as a main component, drugs such as nitroglycerin, isosorbide dinitrate, estradiol, scopolamine, clonidine, nicotine, testosterone, fentanyl, tulobuterol etc., have been developed and marketed, and studies on the transdermal drug delivery system using other drugs have been extensively conducted.

Such a transdermal drug delivery system has lots of advantages compared to conventional oral administration or injection. For example, orally administered drug is absorbed in gastrointestinal tract and before entering systemic circulation, converted to inactive metabolite due to first-pass effect by which drug is metabolized by enzymes in liver, resulting in reduction of efficacy. In particular, nonsteroidal anti-inflammatory drugs, upon oral administration, frequently cause gastric disorder, but by transdermal administration, the first-pass effect can be avoided and the degree and occurrence of gastric disorder can be reduced. Further in the case of the transdermal drug delivery system, blood level of drug can be maintained at constant level for a long time, therefore adverse effect, such as it appears in case of oral dosage or injection due to excessively high blood level of drug immediately after the administration, can be prevented, and for the drug that should be administered frequently owing to its short half life, its administration frequency can be reduced. Furthermore, transdermal drug delivery system has an additional advantage that application can be instantly stopped when adverse effect occurs.

However, skin covering the surface of body functions as a barrier to introduction of external substance into body, thus in drug permeation process, as skin itself acts as rate-limiting step, permeability of drug cannot but be restricted. Therefore, except extremely small number of drugs such as scopolamine and nitroglycerin, in most of the drugs, percutaneous absorption of an amount of the drug enough for exhibition of pharmacological effect is difficult. Further, it is known that hydrophilic or salt-form drug is difficult to be absorbed through skin due to its high polarity, compared to general drugs.

To overcome this problem, a method of increasing the amount of drug partitioned into skin from transdermal drug delivery system by raising drug concentration within the transdermal drug delivery system, a method of hydrating the skin by using material with low moisture permeation to prevent release of sweat or moisture, a method of using permeation enhancers which reduce the barrier function against external substance and a method of using ultrasound or electric current have been suggested and extensively studied.

On the other hand, with regard to drugs applied to transdermal preparation, hydrophilic or salt-form drugs have no good compatibility with adhesive base and show inferior skin permeation relative to hydrophobic or base drug, thus hydrophobic or base drugs have been mainly used.

However, the reason of converting drug into salt form lies in solubilization of drug with very low solubility, masking of bad taste and smell, solidification of liquid drug or improvement of drug stability. Therefore, in case of base drug with bad smell or low stability, it is difficult to develop transdermal drug delivery system.

In addition, adhesives generally used for transdermal medication and patch should have adhesive property enough for attachment of the preparation to skin for a long time. As a platform, acryl, rubber or silicone pressure-sensitive adhesive can be enumerated. Among them, rubber and silicone adhesive are basically hydrophobic compared to acrylic adhesive, thus as an adhesive for hydrophilic or salt-form drug, acrylic adhesive is suitable.

However, among the acrylic adhesives, in case of nearly hydrophilic, emulsion-type acrylic adhesives, mixing with excessive amount of salt-form drug breaks emulsion state, causing precipitation of adhesive. Therefore, it is difficult to allow sufficient amount of drug for exhibition of its pharmacological effect to be loaded, leading to difficulty in preparing transdermal patch.

For the purpose of resolving the problem as described above, attempt of adding solubilizer such as polyethylene glycol, glycerin, or of introducing hydrophilic polyvinylpyrrolidone into acrylic adhesive has been made to allow hydrophilic or salt form drug to be loaded in adhesive layer. For example, the following patents can be enumerated.

US Patent No. 5,252,588 describes that eperisone, tolperisone or salt thereof is formulated into transdermal preparation by using acrylic adhesive to which polyvinylpyrrolidone was introduced and by adding cross-linked polyvinylpyrrolidone.

US Patent No. 5,035,894 discloses an adhesive composition comprising a random graft copolymer having a siloxane backbone grafted with polyethylene oxide units. Hydrophilic drugs such as certain steroids can be dispersed in the adhesive composition to form a controlled release composition.

Japanese Patent Laid-open JP7145048A discloses preparation of a transdermal patch of a salt of guanabenz and guanfacine by using N-vinyl-2-pyrrolidone, which is one kind of monomer of acrylic resin, and by adding poly(ethylene glycol) and polyvinylpyrrolidone.

International Patent Application WO200006659A1 is characterized in that drug showing high skin permeability in the presence of moisture is administered via transdermal route by allowing an acrylic adhesive to contain a liquid polyhydric alcohol such as glycerin, propylene glycol, 1,3-butylene glycol, diglycerine, dipropylene glycol, 1,2,6-hexanetriol, sorbitol, polyethylene glycol and pentaerythritol.

However, simple addition of solubilizers causes a change of properties of the acrylic adhesive, resulting in a negative effect to skin adhesion. As most of the solubilizers are hydrophilic, compatibility with acrylic adhesive is not good, thus it is difficult to select an adequate solubilizer. Additionally, excessive use of such solubilizers might cause skin irritation.

### SUMMARY OF THE INVENTION

The inventors of the present invention conducted extensive studies to develop a transdermal preparation with improved skin permeation while including higher concentrations of a hydrophilic or salt form drug, and as the result, completed the present invention based on discovery that the said object can be accomplished by using, as an adhesive for the said preparation, an acrylic adhesive having a poly(ethylene oxide) or poly(ethylene oxide) monomethyl ether side chain.

The objective of the present invention lies in providing a transdermal preparation with improved skin permeation, which can contain higher concentrations of a hydrophilic or salt-form drug, and guarantee drug stability within the preparation.

### BRIEF DESCRIPTION OF THE DRAWINGS

Fig. 1 shows release rate of salt-form drug from the preparations of Example 4 (●) and Comparative Examples 4-1 (◆) and 4-2 (■).
Fig. 2 shows release rate of salt-form drug from the preparations of Example 8 (●) and Comparative Examples 8 (■).

### DETAILED DESCRIPTION OF THE INVENTION

The present invention relates to a transdermal preparation that can contain high concentrations of a hydrophilic or salt-form drug in an acrylic adhesive, shows increased skin permeation of drug, and allows improved drug stability within the adhesive layer. That is, the present invention relates to a transdermal preparation comprising drug to be absorbed through skin and an adhesive that can contain the said drug, wherein said drug is hydrophilic or in salt form, and said adhesive is an acrylic polymer having poly(ethylene oxide) (called poly(ethylene glycol) in case molecular weight is below 10,000) or poly(ethylene oxide) monomethylether at side chain.

The preparation of the present invention can further comprise a solubilizer to increase the drug concentration within the adhesive layer.

The preparation of the present invention can further comprise a skin permeation enhancer to improve skin permeation of the drug within the adhesive layer.

The present invention is characterized by using an acrylic adhesive having poly(ethylene oxide) or poly(ethylene oxide) monomethyl ether at side chain to increase the content of hydrophilic or salt-form drug within base and to raise skin permeation of drug.

According to other aspect of the present invention, the concentration of hydrophilic or salt-form drug in transdermal preparation can be increased by adding solubilizers for the acrylic adhesive. In addition, skin permeation enhancers can be further contained to improve drug permeation into the skin by increasing partition of drug from transdermal preparation into skin or by reducing skin barrier function.

In general, hydrophilic or salt-form drugs have very high solubility to water. Accordingly, their solubility toward conventional acrylic adhesive is substantially low, leading to difficulty to allow sufficient amount of drug for exhibition of pharmacological effect to be contained in acrylic adhesive layer. To overcome such limitation, the inventors of the present invention used acrylic adhesive where monomer having hydrophilic poly(ethylene oxide) or poly(ethylene oxide) monomethyl ether was introduced, thereby to increase skin permeation by improving the solubility of the hydrophilic or salt-form drug toward acrylic adhesive.

Conventional acrylic adhesive can be prepared by copolymerization of monomer, For example, alkyl(meth)acrylate monomer such as methylmethacrylate, methylacrylate, ethylacrylate, propylacrylate, isopropylacrylate, butylacrylate, isobutylacrylate, t-butylacrylate, butylmethacrylate, isobutylmethacrylate, t-butylmethacrylate, 2-ethylhexylacrylate and glycidylmethylacrylate; hydroxyl monomer such as hydroxyethylacrylate and hydroxypropylacrylate; carboxyl monomer such as acrylic acid and methacrylic acid; or monomer such as vinyl acetate.

The acrylic adhesive according to the present invention can be prepared by copolymerization of the monomer as described above in conjunction with monomer providing poly(ethylene oxide), for example, poly(ethylene oxide) monomethacrylate, poly(ethylene oxide) acrylate, or monomer providing poly(ethylene oxide) monomethyl ether, for example, poly(ethylene oxide) monomethyl ether monomethacrylate, or prepared by introducing poly(ethylene oxide) or poly(ethylene oxide) monomethyl ether via a chemical reaction into already-prepared acrylic adhesive, which allows the adhesive to have poly(ethylene oxide) or poly(ethylene oxide) monomethyl ether at side chain.

The molecular weight of poly(ethylene oxide) or poly(ethylene oxide) monomethyl ether supplied by the said monomer and that of poly(ethylene oxide) or poly(ethylene oxide) monomethyl ether introduced into side chain, are in a range of 100-30000, preferably 400-5000, respectively. The amount of poly(ethylene oxide) or poly(ethylene oxide) monomethyl ether in the final product polymer is, against the total weight of polymer, in a range of 0.01-50%, preferably 0.05-30% by weight.

The polymer having, as a side chain, poly(ethylene oxide) or poly(ethylene oxide) monomethyl ether according to the present invention can be obtained by polymerizing at a time, in adequate ratio, poly(ethylene oxide) monomethacrylate, poly(ethylene oxide) acrylate or poly(ethylene oxide) monomethyl ether monomethacrylate, which is provided as a monomer, and conventional acrylates as described above. Or it can be prepared by introduction of poly(ethylene oxide) or poly(ethylene oxide) monomethyl ether into already-prepared acrylic adhesive.

As a hydrophilic or salt-form drug that can be incorporated into the transdermal preparation of the present invention, sodium, potassium or diethylammonium salt of diclofenac, amfenac, aceclofenac or alclofenac; ketorolac tromethamine; hydrochloride, phosphate or methanesulfonate salt of eperisone or tolperisone; oxybutynin chloride; hydrochloride, hydrobromide, fumarate, succinate or tartrate salt of diphenhydramine, ketotifen, doxylamine, promethazine or trimeprazine; hydrochloride or sulfate salt of tulobuterol, clenbuterol, procaterol or terbutaline; hydrochloride salt of ondansetron, granisetron or ramosetron can be enumerated, but not limited to them.

The amount of these drugs in the preparation can be in a range of 1-50% by weight based on the total weight of acrylic adhesive layer, preferably 1-40%, more preferably 2-30% by weight.

In transdermal preparation, which delivers drug through normal skin, the drug should have compatibility with adhesive polymer, In addition, a large amount of drug should be able to be delivered to the skin by migration of drug from the preparation into skin and then the migrated drug should enters the systemic circulation via skin permeation. Only if it is that way, the desired efficacy can be exhibited.

As the skin, which is surface of body, acts as a barrier against introduction of external pathogen or toxic substance into body as described above, limiting permeation or delivery of drug. Thus, preparation based on transdermal drug delivery system frequently uses skin permeation enhancers to increase skin permeation, in particular, permeation through stratum corneum, the upper most barrier to introduction of external substance.

The skin permeation enhancers used in conventional transdermal drug delivery system were usually selected based on search for substance that improves skin permeability only for a drug. Thus, in real preparation, such selected skin permeation enhancer shows no good compatibility with adhesive polymer, or when applied to preparation using transdermal system, frequently fails to exhibit the desired effect, though the effect is exhibited in liquid vehicle upon mixing with drug.

Therefore, to develop more effective transdermal drug delivery system for hydrophilic or salt-form drug, the present invention uses a solubilizer that increases solubility of hydrophilic or salt form drug toward polymer adhesive to enable larger amount of hydrophilic or salt form drug to be solubilized in polymeric adhesive and a skin permeation enhancer that stimulates the migration toward skin and the skin permeation of hydrophilic or salt form drug, alternatively or in combination of them, thereby providing preparation with far superior skin permeation compared to conventional preparation.

As an example of the solubilizer for adhesive layer of hydrophilic or salt-form drug as described above, distilled water, ethanol, isopropanol, propylene glycol, glycerin, poly(ethylene glycol) (in particular, poly(ethylene glycol) of molecular weight of 200-2000), ethoxydiglycol, dimethylsulfoxide can be enumerated and these can be used in a range of 0.5-50 % by weight based on adhesive layer, preferably 1-30% by weight.

In addition, as an example of skin permeation enhancer that improves skin permeability of hydrophilic or salt form drug by decreasing barrier function of the skin, higher fatty acids such as lauric acid and oleic acid; higher alcohols such as lauryl alcohol and oleyl alcohol; higher fatty acid esters such as isopropyl myristate; fatty acid esters of glycerin such as glycerol monolaurate and glycerol monooleate; fatty acid ethers of poly(ethylene glycol) such as polyoxyethylene(2) lauryl ether and polyoxyethylene(2) oleyl ether; fatty acid esters of poly(ethylene glycol) such as poly(ethylene glycol) laurate; fatty acid ethers of propylene glycol such as propylene glycol lauryl ether; fatty acid esters of propylene glycol such as propylene glycol monolaurate and propylene glycol monooleate; sorbitan fatty acid esters such as sorbitan monolaurate and sorbitan monooleate; poly(ethylene glycol) sorbitan fatty acid esters such as poly(ethylene glycol) sorbitan monolaurate; terpenes such as menthol, menthol derivative and limonene; sulfoxides such as dimethyl sulfoxide and dodecyl sulfoxide; pyrrolidones such as N-methyl-2-pyrrolidone; amides such as lauryl diethanolamide; and N-hydroxy methyl lactate, sorbitol, urea, squalene, olive oil, mineral oil and its derivative can be enumerated, and these can be used in a range of 0.5-50% by weight based on adhesive layer, preferably 1-30% by weight.

The transdermal preparation according to the present invention can be formulated into patch, plaster, tape, poultice, etc., and the preparation methods for the each formulation are known to a person skilled in the art.

For example, in case of patch, generally, backing material is used to protect the preparation, and skin permeation, adhesion and appearance can be controlled to some degree by using additional backing material. As the backing material used in the present invention, any backing material used in conventional transdermal system can be used. For example, material with high air and moisture permeability such as non-woven fabric, cotton fabric and woven fabric, or mono-layer film or laminated film of poly(ethylene terephthalate), polyurethane, polyethylene, polypropylene, ethylene vinyl acetate, aluminum-treated polyethylene, etc. can be used. If necessary, non-woven fabric or cotton fabric can be used in laminated form along with plastic film, which does not have moisture permeability.

In the below, the present invention is more specifically explained by Referential Example, Comparative Example and Example.

### Referential Example 1

### (A) Preparation of acrylic adhesive-1 (Comparison)

2-ethylhexylacrylate 40 weight part, acrylic acid 6 weight part, methylmethacrylate 20 weight part, vinyl acetate 34 weight part, benzoyl peroxide (BPO) 1.0 weight part and ethyl acetate 100 weight part were added to a reaction vessel with reflux condenser and stirrer, and polymerization was conducted with slow stirring under nitrogen atmosphere at 60°C. To regulate polymerization degree, ethyl acetate 100 weight part was slowly added to the reaction mixture during the polymerization and the reaction was conducted for 9 hours. At this time, the polymerization degree was at least 99.9%. Self-cross linking product was obtained by adding aluminum acetyl acetonate to the polymerization product under stirring (200 rpm). Adequate amount of ethyl acetate was added to the polymer solution obtained to make the solid content to be about 40% by weight. As the result, as an acrylic adhesive, copolymer consisting of ethylhexylacrylate, acrylic acid, methylmethacrylate and vinylacetate was obtained.

### (B) Preparation of acrylic adhesive-2 (Comparison)

2-ethylhexylacrylate 97.4 weight part, methacrylic acid 2.5 weight part, poly(ethylene glycol) dimethacrylate 0.1 weight part, benzoyl peroxide 1.0 weight part and ethyl acetate 100 weight part were added to a reaction vessel with reflux condenser and stirrer, and polymerization was conducted with slow stirring under nitrogen atmosphere at 60°C. To regulate polymerization degree, ethyl acetate 100 weight part was slowly added to the reaction mixture during the polymerization and the reaction was conducted for 9 hours. At this time, the polymerization degree was at least 99.9%. The resulting mixture was adjusted with addition of ethyl acetate so as to make the solid content to be about 40% by weight. As the result, as an acrylic adhesive, copolymer consisting of 2-ethylhexylacrylate, methacrylate and poly(ethylene glycol) dimethacrylate was obtained

### (C) Preparation of acrylic adhesive-3 (Comparison)

Under the same condition as described in the above Reference Example 1(B), 2-ethylhexylacrylate 85 weight part, vinylpyrrolidone 15 weight part and benzoyl peroxide 0.1 weight part were copolymerized by addition of ethyl acetate. At this time, the polymerization degree was at least 99.9%. Adequate amount of ethyl acetate was added to the polymer solution obtained to make the solid content to be about 40% by weight. As the result, as an acrylic adhesive, copolymer consisting of 2-ethylhexylacrylate and vinylpyrrolidone was obtained.

### (D) Preparation of acrylic adhesive-4 (The Present Invention)

Under the same condition as described in the above Referential Example 1(B), 2-ethylhexylacrylate 65 weight part, poly(ethylene glycol)(400) monomethacrylate 5 weight part (the figure within the parenthesis means the molecular weight of poly(ethylene glycol), and same in the below), butylacrylate 20 weight part, methylmethacrylate 10 weight part and benzoyl peroxide 0.1 weight part were copolymerized by addition of ethyl acetate. At this time, polymerization degree was at least 99.9%. Adequate amount of ethyl acetate was added to the polymer solution obtained to make the solid content to be about 40% by weight. In this way, acrylic adhesive having poly(ethylene oxide) as a side chain was obtained.

### (E) Preparation of acrylic adhesive-5 (The Present Invention)

Except for using poly(ethylene glycol)(1000) monomethacrylate instead of poly(ethylene glycol)(400) monomethacrylate, it was prepared according to the procedure employed in the Reference Example 1(D). At this time, polymerization degree was at least 99.9%. Adequate amount of ethyl acetate was added to the polymer solution obtained to make the solid content to be about 40% by weight. As the result, acrylic adhesive having poly(ethylene oxide) as a side chain was obtained.

### (F) Preparation of acrylic adhesive-6 (The Present Invention)

2-ethylhexylacrylate 60 weight part, poly(ethylene glycol)(400) acrylate 10 weight part, hydroxyethylacrylate 5 weight part, methylmethacrylate 10 weight part, acrylic acid 5 weight part, vinyl acetate 10 weight part and benzoyl peroxide 0.1 weight part were copolymerized by addition of ethyl acetate under the same condition as described in the Referential Example 1(D). At this time, the polymerization degree was at least 99.9%. Adequate amount of ethyl acetate was added to the polymer solution obtained to make the solid content to be about 40% by weight. As the result, acrylic adhesive having poly(ethylene oxide) as a side chain was obtained.

### (G) Preparation of acrylic adhesive-7 (The Present Invention)

2-ethylhexylacrylate 50 weight part, poly(ethylene glycol)(400) monomethylether monomethacrylate 10 weight part, butylacrylate 15 weight part, methylmethacrylate 10 weight part, vinyl acetate 5 weight part, acrylic acid 5 weight part, hydroxyethylacrylate 5 weight part and benzoyl peroxide 0.1 weight part were copolymerized by addition of ethyl acetate under the same condition as described in the Referential Example 1(D). At this time, the polymerization degree was at least 99.9%. Adequate amount of ethyl acetate was added to the polymer solution obtained to make the solid content to be about 40% by weight. As the result, acrylic adhesive having poly(ethylene oxide) monomethyl ether as a side chain was obtained.

### (H) Preparation of acrylic adhesive-8 (The Present Invention)

Except for using poly(ethylene glycol)(1000) monomethylether monomethacrylate instead of poly(ethylene glycol)(400) monomethylether monomethacrylate, it was prepared according to the procedure employed in the Reference Example 1(G). At this time, polymerization degree was at least 99.9%. Adequate amount of ethyl acetate was added to the polymer solution obtained to make the solid content to be about 40% by weight. In this way, acrylic adhesive having poly(ethylene oxide) monomethyl ether as a side chain was obtained.

### (I) Preparation of acrylic adhesive-9 (The Present Invention)

2-ethylhexylacrylate 40 weight part, acrylic acid 6 weight part, methylmethacrylate 20 weight part, vinyl acetate 34 weight part, benzoyl peroxide (BPO) 1.0 weight part and ethyl acetate 100 weight part were put into a reaction vessel with reflux condenser and stirrer, and polymerization was carried out with slow stirring under nitrogen atmosphere at 60°C. To regulate the polymerization degree, ethyl acetate 100 weight part was slowly added to the reaction mixture during the polymerization reaction and the reaction was carried out for 9 hours. The synthesis product was precipitated in excess amount of methanol and dried in vacuum oven at 60°C for 2 days. At this time, the polymerization degree was at least 99.9%. The synthesized acrylic adhesive 80 weight part, poly(ethylene glycol)(400) monomethylether 20 weight part, p-toluenesulfonic acid 0.5 weight part, hydrochloric acid 0.5 weight part and tetrahydrofuran(THF) 200 weight part were added to a reaction vessel with reflux condenser and stirrer, and the reaction was conducted with stirring under nitrogen atmosphere at 100°C for 16 hours. The synthesis product was precipitated with excess amount of distilled water and dried in vacuum oven at 60°C for 2 days. Adequate amount of ethyl acetate was added to thus obtained polymer to allow solid content to be about 40% by weight. As the result, acrylic adhesive with poly(ethylene oxide) monomethyl ether as a side chain was obtained.

### Comparative Example 1-1

Diclofenac sodium 1.0g was dissolved in 9.0g (dried weight) of acrylic adhesive-1 prepared in the Referential Example 1(A), coated on release liner so that thickness after drying is to be about 100µm, and dried in oven at 80°C for 20 min. Polyethylene film was laminated on this to prepare a patch.

### Comparative Example 1-2

Diclofenac sodium 2.0g and poly(ethylene glycol)(400) 1.0g were dissolved in 8.0g (dried weight) of acrylic adhesive-1 prepared in the Referential Example 1(A), coated on release liner so that thickness after drying is to be about 50µm, and dried in oven at 80°C for 20 min. Polyethylene film was laminated on this to prepare a patch.

### Example 1

Diclofenac sodium 2.0g was dissolved in 8.0g (dried weight) of acrylic adhesive-4 prepared in the Referential Example 1(D), coated on release liner so that thickness after drying is to be about 50µm, and dried in oven at 80°C for 20 min. Polyethylene film was laminated on this to prepare a patch.

### Comparative Example 2

Ketorolac tromethamine 1.5g, glycerin 1.0g and sorbitan monolaurate 0.5g were dissolved in 7.0g (dried weight) of acrylic adhesive-2 prepared in the Referential Example 1(B), coated on release liner so that thickness after drying is to be about 50µm, and dried in oven at 80°C for 20 min. Polyethylene film was laminated on this to prepare a patch.

### Example 2

Ketorolac tromethamine 2.0g, glycerin 1.0g and sorbitan monolaurate 0.5g were dissolved in 6.5g (dried weight) of acrylic adhesive-5 prepared in the Referential Example 1(E), coated on release liner so that thickness after drying is to be about 40µm, and dried in oven at 80°C for 20 min. Polyethylene film was laminated on this to prepare a patch.

### Comparative Example 3

Eperisone hydrochloride 1.0g and polyplasdone INF-10® (crosslinked polyvinylpyrrolidone) 0.5g were mixed with 8.5g(dried weight) of acrylic adhesive-3 prepared in the Referential Example 1(C) to dissolve eperisone hydrochloride, coated on release liner so that thickness after drying is to be about 50 µm, and then dried in oven at 80°C for 20 min. As the result, polyplasdone INF-10® is uniformly dispersed in fine crystalline state within the adhesive layer. Poly(ethylene terephthalate) film was laminated on this to prepare a patch.

### Example 3

Eperisone hydrochloride 1.0g and distilled water 0.5g were dissolved in 8.5g (dried weight) of acrylic adhesive-4 prepared in the Referential Example 1(D), coated on release liner so that thickness after drying is to be about 50µm, and dried in oven at 80°C for 20 min. Poly(ethylene terephthalate) film was laminated on this to prepare a patch.

### Comparative Example 4-1

Tolperisone hydrochloride 0.5g, ethoxydiglycol 0.5g and polyplasdone INF-10® 0.5g were mixed with 8.5g(dried weight) of acrylic adhesive-2 prepared in the Referential Example 1(B) to dissolve tolperisone hydrochloride, coated on release liner so that thickness after drying is to be about 40 µm, and then dried in oven at 80°C for 20 min. As the result, polyplasdone INF-10® is uniformly dispersed in fine crystalline state within the adhesive layer. Poly(ethylene terephthalate) film was laminated on this to prepare a patch.

### Comparative Example 4-2

Tolperisone hydrochloride 0.5g, ethoxydiglycol 0.5g and polyplasdone INF-10® 0.5g were mixed with 8.5g(dried weight) of acrylic adhesive-3 prepared in the Referential Example 1(C) to dissolve tolperisone hydrochloride, coated on release liner so that thickness after drying is to be about 40 µm, and then dried in oven at 80°C for 20 min. As the result, polyplasdone INF-10® is uniformly dispersed in fine crystalline state within the adhesive layer. Poly(ethylene terephthalate) film was laminated on this to prepare a patch.

### Example 4

Tolperisone hydrochloride 0.5g, ethoxydiglycol 0.5g and distilled water 0.5g were dissolved in 8.5g (dried weight) of acrylic adhesive-4 prepared in the Referential Example 1(D), coated on release liner so that thickness after drying is to be about 40µm, and dried in oven at 80°C for 20 min. Poly(ethylene terephthalate) film was laminated on this to prepare a patch.

### Comparative Example 5

Oxybutynin chloride 1.5g, propylene glycol 0.5g and lauryldiethanol amide 0.5g were dissolved in 7.5g (dried weight) of acrylic adhesive-2 prepared in the Referential Example 1(B), coated on release liner so that thickness after drying is to be about 150µm, and dried in oven at 80°C for 20 min. Polyurethane film was laminated on this to prepare a patch.

### Example 5

Oxybutynin chloride 3.0g, propylene glycol 0.5g and lauryldiethanol amide 0.5g were dissolved in 6.0g (dried weight) of acrylic adhesive-5 prepared in the Referential Example 1(E), coated on release liner so that thickness after drying is to be about 75µm, and dried in oven at 80°C for 20 min. Polyurethane film was laminated on this to prepare a patch.

### Comparative Example 6

Diphenhydramine hydrochloride 0.7g, glycerin 1.0g and N-methyl-2-pyrrolidone 1.0g were dissolved in 7.3g (dried weight) of acrylic adhesive-2 prepared in the Referential Example 1(B), coated on release liner so that thickness after drying is to be about 80µm, and dried in oven at 80°C for 20 min. Polypropylene film was laminated on this to prepare a patch.

### Example 6

Diphenhydramine hydrochloride 1.5g, glycerin 1.0g and N-methyl-2-pyrrolidone 1.0g were dissolved in 6.5g (dried weight) of acrylic adhesive-6 prepared in the Referential Example 1(F), coated on release liner so that thickness after drying is to be about 40µm, and dried in oven at 80°C for 20 min. Polypropylene film was laminated on this to prepare a patch.

### Comparative Example 7

Ketotifen fumarate 1.0g and dimethylsulfoxide 1.0g were dissolved in 8.0g (dried weight) of acrylic adhesive-1 prepared in the Referential Example 1(A), coated on release liner so that thickness after drying is to be about 90µm, and dried in oven at 80°C for 20 min. Polyethylene film was laminated on this to prepare a patch.

### Example 7

Ketotifen fumarate 1.0g and dimethylsulfoxide 1.0g were dissolved in 8.0g (dried weight) of acrylic adhesive-7 prepared in the Referential Example 1(G), coated on release liner so that thickness after drying is to be about 90µm, and dried in oven at 80°C for 20 min. Polyethylene film was laminated on this to prepare a patch.

### Comparative Example 8

Doxylamine succinate 0.3g, poly(ethylene glycol)(400) 1.0g and isopropyl myristate 1.0g were dissolved in 7.7g (dried weight) of acrylic adhesive-2 prepared in the Referential Example 1(B), coated on release liner so that thickness after drying is to be about 90µm, and dried in oven at 80°C for 20 min. Poly(ethylene terephthalate) film was laminated on this to prepare a patch.

### Example 8

Doxylamine succinate 0.6g, poly(ethylene glycol)(400) 1.0g and isopropyl myristate 1.0g were dissolved in 7.4g (dried weight) of acrylic adhesive-8 prepared in the Referential Example 1(H), coated on release liner so that thickness after drying is to be about 45µm, and dried in oven at 80°C for 20 min. Poly(ethylene terephthalate) film was laminated on this to prepare a patch.

### Comparative Example 9

Promethazine hydrochloride 0.5g, propylene glycol 0.5g and polyoxyethylene(2) lauryl ether 1.0g were dissolved in 8.0g (dried weight) of acrylic adhesive-1 prepared in the Referential Example 1(A), coated on release liner so that thickness after drying is to be about 80µm, and dried in oven at 80°C for 20 min. Poly(ethylene terephthalate) film was laminated on this to prepare a patch.

### Example 9

Promethazine hydrochloride 1.0g, propylene glycol 0.5g and polyoxyethylene(2) lauryl ether 1.0g were dissolved in 7.5g (dried weight) of acrylic adhesive-4 prepared in the Referential Example 1(D), coated on release liner so that thickness after drying is to be about 40µm, and dried in oven at 80°C for 20 min. Poly(ethylene terephthalate) film was laminated on this to prepare a patch.

### Comparative Example 10

Trimeprazine tartrate 1.0g, ethoxydiglycol 0.5g and sorbitan monolaurate 1.0g were dissolved in 7.5g (dried weight) of acrylic adhesive-2 prepared in the Referential Example 1(B), coated on release liner so that thickness after drying is to be about 100µm, and dried in oven at 80°C for 20 min. Polyurethane film was laminated on this to prepare a patch.

### Example 10

Trimeprazine tartrate 2.0g, ethoxydiglycol 0.5g and sorbitan monolaurate 1.0g were dissolved in 6.5g (dried weight) of acrylic adhesive-4 prepared in the Referential Example 1(E), coated on release liner so that thickness after drying is to be about 50µm, and dried in oven at 80°C for 20 min. Polyurethane film was laminated on this to prepare a patch.

### Comparative Example 11

Tulobuterol hydrochloride 0.5g and propylene glycol monolaurate 1.0g were dissolved in 8.5g (dried weight) of acrylic adhesive-1 prepared in the Referential Example 1(A), coated on release liner so that thickness after drying is to be about µm, and dried in oven at 80°C for 20 min. Polyethylene film was laminated on this to prepare a patch.

### Example 11

Tulobuterol hydrochloride 1.0g and propylene glycol monolaurate 1.0g were dissolved in 8.0g (dried weight) of acrylic adhesive-6 prepared in the Referential Example 1(F), coated on release liner so that thickness after drying is to be about 40µm, and dried in oven at 80°C for 20 min. Polyethylene film was laminated on this to prepare a patch.

### Comparative Example 12

Except for using clenbuterol hydrochloride instead of tulobuterol hydrochloride, a patch was prepared according to the procedure employed in the Comparative Example 11.

### Example 12

Except for using clenbuterol hydrochloride instead of tulobuterol hydrochloride, a patch was prepared according to the procedure employed in the Example 11.

### Comparative Example 14

Ondansetron hydrochloride 0.8g, ethoxydiglycol 1.0g and polyoxyethylene(2) oleyl ether 1.0g were dissolved in 7.2g (dried weight) of acrylic adhesive-1 prepared in the Referential Example 1(A), coated on release liner so that thickness after drying is to be about 100µm, and dried in oven at 80°C for 20 min. Polyethylene film was laminated on this to prepare a patch.

### Example 14

Ondansetron hydrochloride 1.6g, ethoxydiglycol 1.0g and polyoxyethylene(2) oley lether 1.0g were dissolved in 6.4g (dried weight) of acrylic adhesive-4 prepared in the Referential Example 1(D), coated on release liner so that thickness after drying is to be about 50µm, and dried in oven at 80°C for 20 min. Polyethylene film was laminated on this to prepare a patch.

### Comparative Example 15

Granisetron hydrochloride 0.5g, poly(ethylene glycol))(1000) 1.0g and polyoxyethylene(2) oleyl ether 1.0g were dissolved in 7.5g (dried weight) of acrylic adhesive-1 prepared in the Referential Example 1(A), coated on release liner so that thickness after drying is to be about 100µm, and dried in oven at 80°C for 20 min. Poly(ethylene terephthalate) film was laminated on this to prepare a patch.

### Example 15

Granisetron hydrochloride 1.0g, poly(ethylene glycol)(1000) 1.0g and polyoxyethylene(2) oleyl ether 1.0g were dissolved in 7.0g (dried weight) of acrylic adhesive-9 prepared in the Referential Example 1(I), coated on release liner so that thickness after drying is to be about 50µm, and dried in oven at 80°C for 20 min. Poly(ethylene terephthalate) film was laminated on this to prepare a patch.

### Experimental Example 1: Transdermal permeation test

In male guinea pig weighting 350g, the abdominal hair was cut by hair clipper and completely removed by shaver, and full skin of certain abdominal region was excised and frozen (-20°C or below) until used. For experiment, it was thawed.

The thawed skin was cut into 2X2 cm², and the patch prepared in Comparative Example and Example was cut into 1.5X 1.5 cm² and was attached to stratum corneum.

The skin was positioned within franz-type glass diffusion cell so that the part to which patch was attached faces upward, and distilled water or buffer solution of adequate pH value was placed to the receptor depending on the kind of drugs, and the diffusion cell was maintained at 37°C. Receptor solution (buffer solution) was stirred with constant speed (600 rpm). At a pre-determined time, aliquot of the receptor solution was taken, and the same amount of buffer solution was refilled. Concentration of permeated drug was determined by high pressure liquid chromatography (HPLC). The test result is shown in Table 1.

**[Table 1]**

| Transdermal permeation of hydrophilic or salt-form drugs through guinea pig skin | |
|---|---|
| Patch No. | Cumulative Permeated Amount for 24 hours (µg/cm²) |
| Comparative Example 1-1 | 95.6 ± 10.3 |
| Comparative Example 1-2 | 152.4 ± 14.9 |
| Example 1 | 201.5 ± 21.1 |
| Comparative Example 2 | 43.1 ± 8.8 |
| Example 2 | 77.8 ± 17.4 |
| Comparative Example 3 | 11.6 ± 3.4 |
| Example 3 | 83.1 ± 26.4 |
| Comparative Example 4-1 | 15.4 ± 6.6 |
| Comparative Example 4-2 | 56.3 ± 14.8 |
| Example 4 | 98.2 ± 30.6 |
| Comparative Example 5 | 39.5 ± 8.7 |
| Example 5 | 85.5 ± 16.3 |
| Comparative Example 6 | 83.0 ± 28.2 |
| Example 6 | 141.4 ± 45.0 |
| Comparative Example 7 | 7.8 ± 2.1 |
| Example 7 | 16.6 ± 1.8 |
| Comparative Example 8 | 15.7 ± 1.6 |
| Example 8 | 22.2 ± 6.3 |
| Comparative Example 9 | 62.2 ± 6.5 |
| Example 9 | 80.6 ± 19.0 |
| Comparative Example 10 | 29.1 ± 5.6 |
| Example 10 | 81.0 ± 21.8 |
| Comparative Example 11 | 88.8 ± 33.1 |
| Example 11 | 112.4 ± 37.6 |
| Comparative Example 12 | 75.9 ± 22.3 |
| Example 12 | 101.8 ± 21.8 |
| Comparative Example 14 | 20.8 ± 8.3 |
| Example 14 | 35.7 ± 11.2 |
| Comparative Example 15 | 15.7 ± 4.2 |
| Example 15 | 24.3 ± 7.7 |

### Experimental Example 2: Test of drug stability within patch

The prepared patch was put into aluminum pack, filled with nitrogen gas, sealed and stored at 40°C, relative humidity 75% oven. At a pre-determined time, it was opened, drug was extracted and the residual amount of drug was determined by HPLC. The test result is represented in Table 2.

**[Table 2]**

| Drug stability within patch | | | | |
|---|---|---|---|---|
| Patch No. | Residual amount (%) | | | |
| | 1 month | 2 month | 3 month | 6 month |
| Comparative Example 4-1 | 95.3 | 92.8 | 90.2 | 75.7 |
| Comparative Example 4-2 | 97.6 | 95.9 | 92.8 | 91.0 |
| Example 4 | 98.6 | 102.3 | 99.3 | 99.5 |
| Comparative Example 7 | 94.3 | 89.1 | 86.7 | 82.3 |
| Example 7 | 99.1 | 96.8 | 94.1 | 92.5 |
| Comparative Example 11 | 93.0 | 85.5 | 80.4 | 66.0 |
| Example 11 | 100.9 | 94.3 | 92.1 | 90.3 |

### Experimental Example 3: Drug release test

Drug release test on the prepared patch was conducted according to paddle over disk test method for transdermal delivery systems described in DRUG RELEASE of US Pharmacopoeia 24^{th} ed.

As release solution, depending on the kind of drug, distilled water or adequate buffer solution 500 ml was added to release test vessel, and temperature was maintained at 32.0 ±0.5°C, paddle speed was maintained at 100rpm.

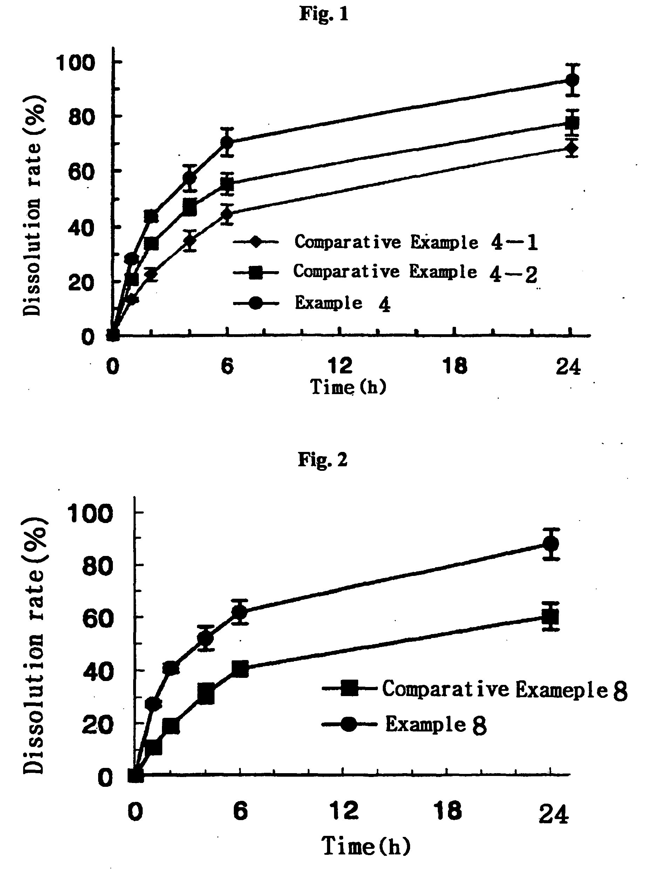

After the start, aliquot of 1 ml was taken by interval of 1hr, 4hr or 24hr and the amount of drug released was determined by HPLC. Whenever aliquot was taken, same amount of buffer solution warmed to 32.0 ± 0.5°C was refilled. The result is illustrated in Fig 1. By the same method, the patches prepared in Example 8 and Comparative Example 8 were tested and the result is illustrated in Fig 2.

As can be seen in Table 1 and Figs. 1 and 2, transdermal permeation rate and release rate of salt-form drug was confirmed to be higher in the Example according to the present invention compared to Comparative Example.

As can be seen in Table 2, drug stability in Examples 4, 7 and 11 according to the present invention was superior to those in Comparative Examples 4-1, 4-2, 7 and 11.

The transdermal preparation of the present invention as explained above can contain effective concentration of hydrophilic or salt-form drug, accomplishes superior transdermal permeation and drug stability within preparation, by using the acrylic adhesive having poly(ethylene oxide) or poly(ethylene oxide) monomethyl ether as a side chain.

## Claims

1. A transdermal preparation comprising a hydrophilic or salt-from drug to be delivered through skin and an adhesive, **characterized in that** said adhesive is an acrylic adhesive having a poly(ethylene oxide) or poly(ethylene oxide) monomethyl ether side chain.

2. The transdermal preparation according to Claim 1, comprising one or more components selected from solubilizers and skin permeation enhancers.

3. The transdermal preparation according to Claim 1 or 2, wherein the content of the said drug is in a range of 1-50% by weight based on the total weight of the adhesive layer.

4. The transdermal preparation according to Claim 1 or 2, wherein the molecular weight of said poly(ethylene oxide) or poly(ethylene oxide) monomethyl ether is in a range of 100-30000, and the content thereof is in a range of 0.01-50% by weight based on the total weight of polymeric adhesive.

5. The transdermal preparation according to Claim 4, wherein the molecular weight of said poly(ethylene oxide) or poly(ethylene oxide) monomethyl ether is in a range of 400-5000, and the content thereof is in a range of 0.05-30 % by weight based on the total weight of polymeric adhesive.

6. The transdermal preparation according to Claim 1 or 2, wherein said drug is selected from sodium, potassium and diethylammonium salts of diclofenac, amfenac, aceclofenac and alclofenac; ketorolac tromethamine; hydrochloride, phosphate and methanesulfonate salts of eperisone and tolperisone; oxybutynin chloride; hydrochloride, hydrobromide, fumarate, succinate and tartrate salts of diphenhydramine, ketotifen, doxylamine, promethazine and trimeprazine; hydrochloride and sulfate salts of tulobuterol, clenbuterol, procaterol and terbutaline; hydrochloride salts of ondansetron, granisetron and ramosetron.

7. The transdermal preparation according to Claim 2, wherein the solubilizer is at least one component selected from ethanol, isopropanol, poly(ethylene glycol), ethoxydiglycol, distilled water, propylene glycol, glycerin and dimethylsulfoxide, and the content is in a range of 0.5-50% by weight based on the total weight of the adhesive layer.

8. The transdermal preparation according to Claim 2, wherein the skin permeation enhancer is at least one component selected from higher fatty acids; higher alcohols; higher fatty acid esters; fatty acid esters of glycerin; fatty acid ethers of poly(ethylene glycol); fatty acid esters of poly(ethylene glycol); fatty acid ethers of propylene glycol; fatty acid esters of propylene glycol; sorbitan fatty acid esters; poly(ethylene glycol) sorbitan fatty acid esters; terpenes; sulfoxides; pyrrolidones; amides; and N-hydroxy methyl lactate, sorbitol, urea, squalene, olive oil, mineral oil and its derivatives, and the content is in a range of 0.5-50% by weight based on the total weight of the adhesive layer.

9. The transdermal preparation according to Claim 8, wherein the skin permeation enhancer is at least one component selected from lauric acid, oleic acid, lauryl alcohol, oleyl alcohol, glycerol monolaurate, glycerol monooleate, polyoxyethylene(2) lauryl ether, polyoxyethylene(2) oleyl ether, propylene glycol monolaurate, propylene glycol monooleate, sorbitan monolaurate, sorbitan monooleate, lauryl diethanolamide, N-methyl-2-pyrrolidone and isopropyl myristate.

10. The transdermal preparation according to Claims 7 to 9, wherein the content of said solubilizer and said skin permeation enhancer each is in range of 1-30% by weight based on the total weight of the adhesive layer.

## Patentansprüche

1. Transdermales Präparat, umfassend ein hydrophiles Arzneimittel oder eine Salzform eines Arzneimittels, das durch die Haut zu verabreichen ist, und ein Klebstoff, **dadurch gekennzeichnet, dass** das Klebstoff ein Acrylklebstoff mit einer Poly(ethylenoxid)- oder Poly(ethylenoxid)monomethylether-Seitenkette ist.

2. Transdermales Präparat nach Anspruch 1, umfassend eine oder mehrere Komponenten ausgewählt aus Solubilisierungsmitteln und Hautdurchdringungsverbesserungsmitteln.

3. Transdermales Präparat nach Anspruch 1 oder 2, bei dem der Gehalt des Arzneimittels im Bereich von 1 bis 50 Gew.-% auf Basis des Gesamtgewichts der Klebstoffschicht liegt.

4. Transdermales Präparat nach Anspruch 1 oder 2, bei dem das Molekulargewicht des Poly(ethylenoxids) oder Poly(ethylenoxid)monomethylethers im Bereich von 100 bis 30.000 liegt und der Gehalt davon im Bereich von 0,01 bis 50 Gew.-% auf Basis des Gesamtgewichts des polymeren Klebstoffs ist.

5. Transdermales Präparat nach Anspruch 4, bei dem das Molekulargewicht des Poly(ethylenoxids) oder Poly(ethylenoxid)monomethylethers im Bereich von 400 bis 5.000 liegt und der Gehalt davon im Bereich von 0,05 bis 30 Gew.-% auf Basis des Gesamtgewichts des polymeren Klebstoffs ist.

6. Transdermales Präparat nach Anspruch 1 oder 2, bei dem das Arzneimittel ausgewählt ist aus Natrium-, Kalium- und Diethylammoniumsalzen von Diclofenac, Amfenac, Aceclofenac und Alclofenac; Ketorolactromethamin; Hydrochlorid-, Phosphat- und Methansulfonatsalzen von Eperison und Tolperison; Oxybutininchlorid; Hydrochlorid-, Hydrobromid-, Fumarat-, Succinat- und Tartratsalzen von Diphenhydramin, Ketotifen, Doxylamin, Promethazin und Trimeprazin; Hydrochlorid- und Sulfatsalzen von Tulobuterol, Clenbuterol, Procaterol und Terbutalin; Hydrochloridsalzen von Ondansetron, Granisetron und Ramosetron.

7. Transdermales Präparat nach Anspruch 2, bei dem das Solubilisierungsmittel mindestens eine Komponente ausgewählt aus Ethanol, Isopropanol, Poly-(ethylenglycol), Ethoxydiglycol, destilliertem Wasser, Propylenglycol, Glycerin und Dimethylsulfoxid ist und der Gehalt im Bereich von 0,5 bis 50 Gew.-% auf Basis des Gesamtgewichts der Klebstoffschicht liegt.

8. Transdermales Präparat nach Anspruch 2, bei dem das Hautdurchdringungsverbesserungsmittel mindestens eine Komponente ausgewählt aus höheren Fettsäuren; höheren Alkoholen, höheren Fettsäureestern; Fettsäureestern von Glycerin; Fettsäureethern von Poly(ethylenglycol); Fettsäureestern von Poly(ethylenglycol); Fettsäureethern von Propylenglycol; Fettsäureestern von Propylenglycol; Sorbitanfettsäureestern; Poly(ethylenglycol)sorbitanfettsäureestern; Terpenen; Sulfoxiden; Pyrrolidonen; Amiden; und N-Hydroxymethyllactat, Sorbit, Harnstoff, Squalen, Olivenöl, Mineralöl und deren Deriven ist und der Gehalt im Bereich von 0,5 bis 50 Gew.-% auf Basis des Gesamtgewichts der Klebstoffschicht liegt.

9. Transdermales Präparat nach Anspruch 8, bei dem das Hautdurchdringungsverbesserungsmittel mindestens eine Komponente ausgewählt aus Laurinsäure, Ölsäure, Laurylalkohol, Oleylalkohol, Glycerinmonolaureat, Glycerinmonooleat, Polyoxyethylen(2)laurylether, Polyoxyethylen(2)oleylether, Propylenglycolmonolaurat, Propylenglycolmonooleat, Sorbitanmonolaurat, Sorbitanmonooleat, Lauryldiethanolamid, N-Methyl-2-pyrrolidon und Isopropylmyristat ist.

10. Transdermales Präparat nach den Ansprüchen 7 bis 9, bei dem der Gehalt des Solubilisierungsmittels und des Hautdurchdringungsverbesserungsmittels jeweils im Bereich von 1 bis 30 Gew.-% auf Basis des Gesamtgewichts der Klebstoffschicht liegt.

## Revendications

1. Préparation transdermique comprenant un médicament, hydrophile ou sous forme de sel, à administrer à travers la peau, et un adhésif, **caractérisée en ce que** ledit adhésif est un adhésif acrylique comportant, en chaîne latérale, un poly(oxyéthylène) ou un éther monométhylique de poly(oxyéthylène).

2. Préparation transdermique conforme à la revendication 1, comprenant un ou plusieurs composants choisis parmi les agents de solubilisation et les agents favorisant la perméation à travers la peau.

3. Préparation transdermique conforme à la revendication 1 ou 2, dans laquelle la proportion dudit médicament vaut de 1 à 50 % en poids rapporté au poids total de la couche d'adhésif.

4. Préparation transdermique conforme à la revendication 1 ou 2, dans laquelle la masse molaire dudit poly(oxyéthylène) ou éther monométhylique de poly(oxyéthylène) vaut de 100 à 30 000, et sa proportion vaut de 0,01 à 50 % en poids rapporté au poids total d'adhésif polymère.

5. Préparation transdermique conforme à la revendication 4, dans laquelle la masse molaire dudit poly(oxyéthylène) ou éther monométhylique de poly(oxyéthylène) vaut de 400 à 5 000, et sa proportion vaut de 0,05 à 30 % en poids rapporté au poids total d'adhésif polymère.

6. Préparation transdermique conforme à la revendication 1 ou 2, dans laquelle ledit médicament est choisi parmi les sels de sodium, potassium ou diéthylammonium de diclofenac, amfenac, aceclofenac et alclofenac, le kétorolac trométhamine, les sels chlorhydrates, phosphates et méthanesulfonates d'épérisone et de tolpérisone, le chlorure d'oxybutynine, les sels chlorhydrates, bromhydrates, fumarates, succinates et tartrates de diphénhydramine, kétotifène, doxylamine, prométhazine et triméprazine, les sels chlorhydrates et sulfates de tulobutérol, clenbutérol, procatérol et terbutaline, et les sels chlorhydrates de granisétron, ondansétron et ramosétron.

7. Préparation transdermique conforme à la revendication 2, dans laquelle ledit agent de solubilisation est au moins un composant choisi parmi les éthanol, isopropanol, polyéthylèneglycol, éthoxydiglycol, eau distillée, propylèneglycol, glycérol et diméthylsulfoxyde, et se trouve en une proportion de 0,5 à 50 % en poids rapporté au poids total de la couche d'adhésif.

8. Préparation transdermique conforme à la revendication 2, dans laquelle ledit agent favorisant la perméation à travers la peau est au moins un composant choisi parmi les acides gras supérieurs, alcools supérieurs, esters d'acides gras supérieurs, esters d'acides gras et de glycérol, éthers d'alcools dérivés d'acides gras et de polyéthylèneglycol, esters d'acides gras et de polyéthylèneglycol, éthers d'alcools dérivés d'acides gras et de propylèneglycol, esters d'acides gras et de propylèneglycol, esters d'acides gras et de sorbitanne, esters d'acides gras et de sorbitanne polyéthoxylé, terpènes, sulfoxydes, pyrrolidones et amides et les N-hydroxyméthyl-lactate, sorbitol, urée, squalène, huile d'olive, huile minérale et leurs dérivés, et se trouve en une proportion de 0,5 à 50 % en poids rapporté au poids total de la couche d'adhésif.

9. Préparation transdermique conforme à la revendication 8, dans laquelle ledit agent favorisant la perméation à travers la peau est au moins un composant choisi parmi l'acide laurique, l'acide oléique, l'alcool laurylique, l'alcool oléylique, le monolaurate de glycérol, le monooléate de glycérol, le polyoxyéthylène(2)-lauryl-éther, le polyoxyéthylène(2)-oléyl-éther, le monolaurate de propylèneglycol, le monooléate de propylèneglycol, le monolaurate de sorbitanne, le monooléate de sorbitanne, le N,N-bis(hydroxyéthyl)lauramide, la N-méthyl-2-pyrrolidone et le myristate d'isopropyle.

10. Préparation transdermique conforme aux revendications 7 à 9, dans laquelle ledit agent de solubilisation et ledit agent favorisant la perméation à travers la peau se trouvent chacun en une proportion de 1 à 30 % en poids rapporté au poids total de la couche d'adhésif.
